# EUROPEAN PATENT SPECIFICATION

(11) **EP 2 486 923 B1**
(45) Date of publication and mention of the grant of the patent: **09.09.2015**
(21) Application number: 11154114.0
(22) Date of filing: 11.02.2011
(51) Int. Cl.: A61K 31/277, C07C 255/60, C07C 259/06, C07D 249/06, C07D 277/46, A61K 31/165, A61K 31/4192, A61K 31/426, A61P 35/00

(54) **Histone deacetylase (HDAC) inhibiting compounds and method of making same**
Histon-Deacetylase (HDAC) hemmende Verbindungen und Verfahren zu deren Herstellung
Composés inhibant l'histone désacétylase (HDAC) et leur procédé de fabrication

(43) Date of publication of application: 15.08.2012
(73) Proprietor: Dr. Felix Jäger und Dr. Stefan Drinkuth Laborgemeinschaft OHG, 34587 Felsberg-Altenburg (DE); Hochschule Darmstadt, 64295 Darmstadt (DE)
(72) Inventor: Jäger, Felix, Dr., 37079 Göttingen (DE); Drinkuth, Stefan, Dr., 34225 Baunatal (DE); Ludwig, Joachim, Dr., 34123 Kassel (DE); Meyer-Almes, Franz-Josef, Dr., 64853 Otzberg-Lengfeld (DE); Henkes, Leonhard, 64683 Einhausen (DE)
(74) Representative: Stüven, Ralf

(56) References cited:
- EP-A1- 0 454 444
- EP-A1- 1 541 549
- WO-A1-03/070726
- WO-A1-2007/002441
- WO-A2-02/28841
- CN-A- 1 955 167
- US-A- 5 614 214
- US-A1- 2003 149 050
- US-A1- 2005 277 652
- US-A1- 2006 167 317
- US-A1- 2006 183 772
- US-A1- 2006 280 746
- US-A1- 2007 197 605
- DATABASE CAPLUS [Online] CHEMICAL ABSTRACTS SERVICE, COLUMBUS, OHIO, US; VEHLOW, KATI ET AL: "Trialkoxysilyl-functionalized silver(I) carboxylates: New building blocks for the synthesis of immobilizable and immobilized homogeneous catalysts", XP002645239, retrieved from STN Database accession no. 2005:619657 & EUROPEAN JOURNAL OF INORGANIC CHEMISTRY , (13), 2727-2736 CODEN: EJICFO; ISSN: 1434-1948, 2005,
- DATABASE CAPLUS [Online] CHEMICAL ABSTRACTS SERVICE, COLUMBUS, OHIO, US; EISENHUTH, RALF ET AL: "Convenient Syntheses of 3'-Amino-2',3'-dideoxynucleosides, Their 5'-Monophosphates, and 3'-Amino-terminal Oligodeoxynucleotide Primers", XP002645240, retrieved from STN Database accession no. 2008:1411332 & JOURNAL OF ORGANIC CHEMISTRY , 74(1), 26-37 CODEN: JOCEAH; ISSN: 0022-3263, 2009,

## Description

### Technical Field

The invention relates to novel HDAC inhibitors and a method of making such HDAC inhibitors. Further, the invention relates to pharmaceutical compositions comprising the HDAC inhibitors of the invention.

### Background art

Today, cancer is still one of the most threatening diseases in common societies. 13 % of the worldwide deaths (7.9 million people) in 2007 were caused by cancer. As the second most common cause of death (USA), exceeded only by heart diseases, cancer remains a burden to the health and even the funds off many societies ^{[1][2][3]}.

The start of a cancer disease can be caused by environmental influences or the incorporation of toxins into a healthy cell, leading to several damages in its DNA. Cancer cells are characterized by their out-of-control growth, their invasion into different tissues and the extensive spreading over the whole body thru bloodstream and lymph system migration.

Conventionally, cancer is treated by a mixture of chemotherapy, surgery and radiation, depending on the individual type of cancer. For a long time DNA crosslinking agents like Cisplatin were applied in treatments with chemotherapy to induce apoptosis.

During the last decades of cancer research several new targets were explored, leading to new approaches for cancer treatments. Among these targets, the enzyme class of the so called histone deacetylases (HDACs) is one of the most promising ones.

In the nucleus of cells, the DNA is structurally tensely packed by forming nucleosomes consisting of multiple units of a protein octamer and attached convoluted DNA. Those proteins, called histones, have a strong influence on the accessibility for transcriptional proteins by binding the DNA via acetylated histone-lysine residues. Acetylated lysine residues form tense structures with the negatively charged backbone of the DNA leading to reduced gene expression.

HDACs play a prominent role in controlling the acetylation state of lysine residues located at the ends of histones. Occurring as a natural substrate, acetylated lysins become deacetylated by HDACs resulting in reduced gene expression due to a less accessible DNA structure. The loss of acetylations can be restored by the antagonistic histone-acetyl-transferases (HATs). In consequence of their fundamental influence on regulating gene expression, HDACs are associated with several diseases and vice versa are valid targets for cancer treatments ^{[4]}.

In humans, the overall family of HDAC proteins contains 18 different members separated into four classes by their homology to yeast proteins, cellular localization and the structure of their active sites. Class I comprises of HDAC-1, -2, -3 and -8 which are homologue to the yeast protein RPD3 and retain mostly located in the nucleus. Class II HDAC members are divided into the further subclasses IIa and IIb. Class IIa Includes HDAC-4, -5, -7 and -9 which are homologue to yeast HDA1-deacetylase and contain one active site. In contrast, the HDAC IIb members HDAC-6 and -10 contain two active sites. However HDAC-10 carries only one completely functional active site while the second c-terminal localized domain lacks important residues of the active site. Class IIa members have the possibility of shuttling between cytoplasm and the nucleus. In contrast HDAC IIb members are localized in the cytoplasm. The Class IV member HDAC-11 shares a similar catalytic domain with Class I and II members. Yet, no substrate could be identified for this protein. All Class I, II and IV members are zinc dependent deacetylases. In contrast Class III proteins are NAD⁺ dependent and denoted as Sirtuins due to their yeast homologue SirT2 ^{[5][6][7]}.

Despite their strong structural similarities, HDACs are involved in different non-redundant processes, which are tissue specific. Knock-out of HDAC-1, -2, -3 or -7 in mice are lethal in early embryonic states due to aberrant angiogenesis or cell cycle control. Mice with knock-out for HDAC-4, -5, -6, or -9 are viable with occurring abnormalities in cardiovascular, bone and muscle development. In cancer tissue a single knock-down of one HDAC results in specific symptoms. For example, HDAC-2 knock-down in colon cancer cells induces growth arrest, while lagging this effect in osteosarcoma or breast-cancer. Conducted studies found high expression levels of class I HDACs in many primary human cancer cell lines (breast, pancreas, lung, esophageal, gastric, colon, ovary and thyroid). Yet, less is known about expression levels of class II HDACs in cancer cell lines ^{[6][8]}.

The high expression values of different HDAC members in cancer cell lines and their strong influence on gene regulation led to a new approach for cancer treatments based on Histone deacetylase inhibitors (HDACi's). In the last few years several types of HDACi's could be identified for the zinc dependent HDAC classes (I, II and IV). Four main classes are established till today: hydroxamates, cyclic peptides, small fatty acids and benzamidines.
EP 1 541 549 A1, for example, describes tricyclic hydroxamate and benzaminde derivatives as HDAC inhibitors.

Off these four classes, the hydroxamates are the most enlightened group until today. In general, their structure consists of a variable cap group and a metal-binding hydroxamic acid group. Both parts are connected through an aliphatic or aromatic linker ^{[15]}. This structure basically mimics the natural substrate which complexes the zinc in the active site with an acetyl group instead of the hydroxamic acid. One of the first potent hydroxamate HDACi's was isolated from *Streptomyces bydroscopicus*: Trichostatin A (TSA, see below for structures). Based on this structure, a second very potent HDACi got synthesized: Vorinostat (SAHA, see below for structure). SAHA was one of the first HDACi that passed all clinical trials and was applied for treatment of cutaneous T-cell lymphoma (CTCL).

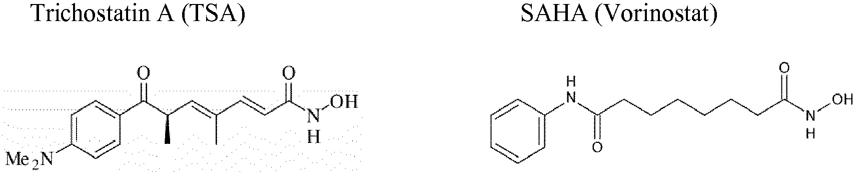

However, most of the very potent HDACi's (TSA, SAHA) are rather unselective inhibitors. The inhibition of several or nearly all HDACs goes along with several side effects. To avoid these pharmacological issues research for HDAC isoform specific inhibitors became an urgent challenge. With the help of high troughput screenings (HTS) several new inhibitor structures were identified. Another way of finding isoform selective inhibitors included the chemical modification of already existing inhibitors, like the hydroxamates. Based on the structure of SAHA and TSA several different cap group modifications altered the selectivity for class I HDACs. In contrast, variations of the linker and metal-binding group had a much lesser influence on the isoform selectivity due to the strong structural homology of all isoforms in their active site structure (interacting with metal-binding group) and in their hydrophobic cavity (interacting with linker).

### Brief Summary of the invention

There still is a need for alternative HDAC inhibitors. Such alternative HDAC inhibitors may, for example, be more specific and/or associated with fewer side effects. It is therefore an object of the invention to provide alternative HDAC inhibitors, in particular more selective, e.g HDAC class II selective HDAC inhibitors. A further object of the invention is to provide a method of making such alternative HDAC inhibitors. Still another object of the invention is the provision of pharmaceutically compositions comprising such alternative HDAC inhibitors.

The present invention provides novel HDAC inhibitors having a perfluorinated linker between the metal(zinc)-binding group and the cap group.

In the attempt to synthesize new HDACi's, the zinc binding groups and different capping structures have been varied, but the spacer was almost exclusively an alkyl chain of different lengths. Due to the exceptional high electronegativity of fluorine atoms perfluorinated spacers have not been taken in consideration for more than 20 years of HDACi synthesis. The only occurrence of fluorine atoms took place in a special class of HDACi inhibitors, the electrophilic ketones, particularly trifluoromethyl ketones, in which the electron attracting property of fluorine atoms is exploited.

Therefore, it was not expected, that compounds with perfluorinated alkyl spacer turned out to be potent inhibitors of HDAH and HDACs. The provision of an HDACi with a perfluorinated linker structure is an innovative step paving the road for novel lead structures in HDACi research.

The HDAC inhibitors according to the present invention are especially useful as an active ingredient in a medicament, in particular a medicament for treating cancer in humans.

### Detailed description of the invention

In one aspect the present invention relates to a novel histone deacetylase (HDAC) inhibiting compound of formula (I) 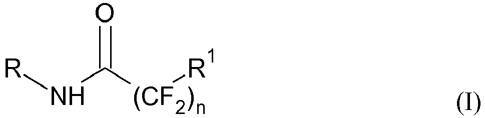 or a pharmaceutically acceptable salt thereof, wherein
n is 4, 5, 6, 7, 8, 9, 10, 11, 12, 13 or 14,
R is a cyclic or acyclic, substituted or unsubstituted, branched or unbranched aliphatic; a cyclic or acyclic, substituted or unsubstituted, branched or unbranched heteroaliphatic; a substituted or unsubstituted, branched or unbranched acyl; substituted or unsubstituted, branched or unbranched aryl; a substituted or unsubstituted, branched or unbranched heteroaryl, and
R¹ is a metal binding group capable of binding a metal ion within an active site of said histone deacetylase.

Preferably, n is 4, 5, 6, 7 or 8, further preferred 5, 6 or 7, especially preferred 6.

Any atom of residue R may further be independently substituted by a hydrogen, a protecting group, an aliphatic moiety, a heteroaliphatic moiety, an acyl moiety; an aryl moiety; a heteroaryl moiety; alkoxy; aryloxy; alkylthio; arylthio; amino, alkylamino, dialkylamino, heteroaryloxy; or heteroarylthio moiety.

Preferably, the metal binding group is a zinc binding group. The metal binding group can, for example, be selected from one of the following groups:

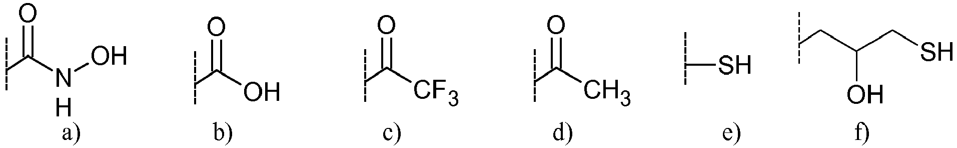

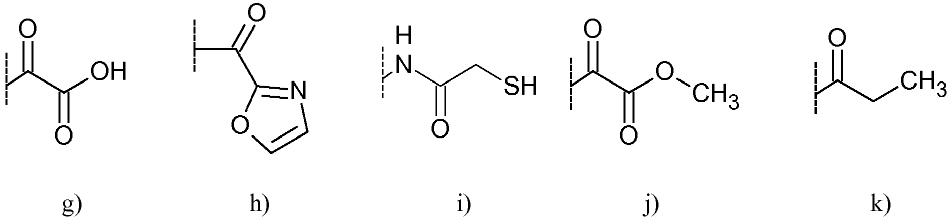

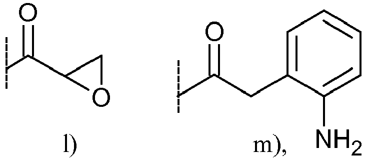

In a further preferred embodiment, the invention provides a compound according to formula I above, wherein
R is 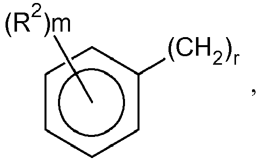 wherein
m is 0, 1, 2, 3, 4or 5
r is 0, 1, 2 or 3, and wherein
R² is hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl.

Thus, the invention provides, for example, a compound according to formula II

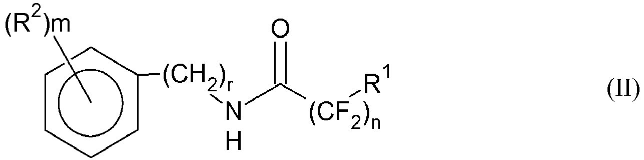

In case of R¹ being a hydroxamic acid group, the invention provides e.g. a compound according to formula IIa:

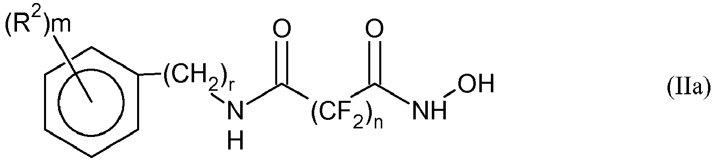

Any atom of residue R² in formula II or IIa above may further be independently substituted by a hydrogen, a protecting group, an aliphatic moiety, a heteroaliphatic moiety, an acyl moiety; an aryl moiety; a heteroaryl moiety; alkoxy; aryloxy; alkylthio; arylthio; amino, alkylamino, dialkylamino, heteroaryloxy; or heteroarylthio moiety.

In a further preferred embodiment, the invention provides a compound according to formula II, wherein R² is one of the following: 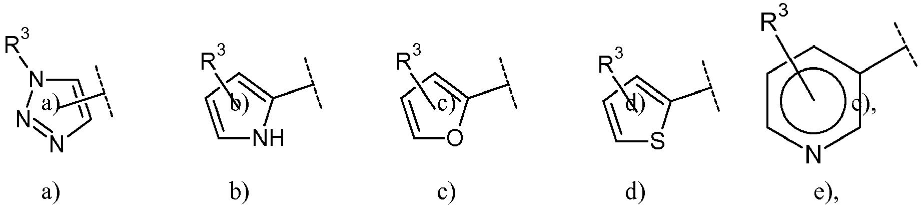 wherein R³ is hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl.

In another preferred embodiment the invention provides a compound according to formula I above, wherein R is one of the following: 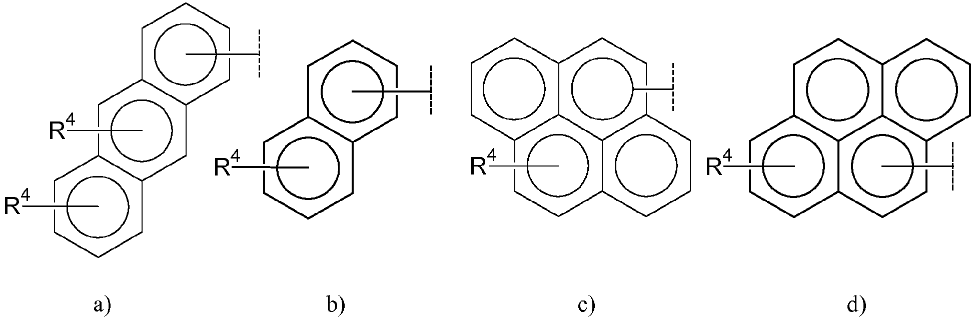 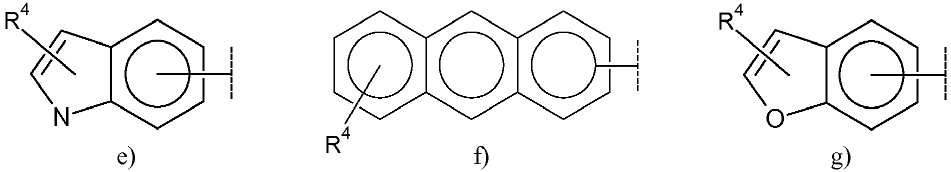 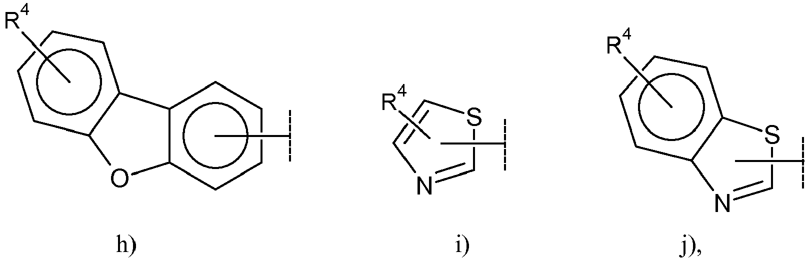 and wherein R⁴ is cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl.

The term "histone deacetylase (HDAC) inhibiting compound" as used herein refers to a class of compounds that interferes with the function of histone deacetylase (HDAC). Histone deacetylases are a class of enzymes removing the acetyl groups from lysine residues in core histones leading to the formation of a condensed and transcriptionally silenced chromatin. The histone deacetylase inhibitors (HDACi's) are a class of cytostatic agents that inhibit the proliferation of tumor cells in culture and in vivo by inducing cell cycle arrest, differentiation and/or apoptosis. In the context of the present invention a compound is considered an HDAC inhibiting compound (or HDAC inhibitor) if the compound exhibits an IC₅₀ of ≤100 µM with at least one HDAC and/or homologous enzyme of humans or other species, preferably an IC₅₀ of ≤80, ≤70, ≤60 or ≤50 µM, and especially preferred an IC₅₀ of ≤40, ≤30, ≤20 or ≤10 µM.

The term "pharmaceutically acceptable salt" as used herein includes acid addition salts, hydrates, alcolates. The acid addition salts may be formed by either strong or weak acids. Representative of strong acids are hydrochloric, sulfuric and phosphoric. Representative of weak acids are fumaric, maleic, succinic, oxalic, citric, tartaric, cyclohexaminic and the like. Non-limiting examples of pharmaceutically acceptable salts are ammonium salts, alkali metal salts such as potassium and sodium (including mono, di- and tri-sodium) salts, alkaline earth metal salts such as calcium and magnesium salts, salts with organic bases such as dicyclohexylamine salts, N-methyl-D-glucamine, and salts with amino acids such as arginine, lysine.

The term "metal binding group" refers to a functional group capable of binding a metal ion within an active site of an enzyme, e.g. histone deacetylase. For example, and most preferred, the metal binding group is a zinc binding group, binding the active site zinc(II) ion of a histone deacetylase. An example for a zinc binding group is the hydroxamic acid group CONHOH.

The term "protecting group" or "protective group", as used herein, refers to a chemical group introduced into a molecule by chemical modification of a functional group in order to obtain chemo selectivity in a subsequent chemical reaction. Examples of such groups are acetyl groups and benzoyl groups.

The term "moiety" means a part of a molecule that may or may not comprise a functional group. Unless otherwise stated, the term is used synonymously to the term "residue"

The term "aliphatic" as used herein refers to acyclic or cyclic, non-aromatic carbon compounds. In aliphatic compounds, carbon atoms are joined together in straight chains, branched chains, or non-aromatic rings (alicyclic compounds). Aliphatic compounds can be saturated, joined by single bonds (alkanes), or unsaturated, with double bonds (alkenes) or triple bonds (alkynes). Besides hydrogen, other elements can be bound to the carbon chain, e.g. oxygen, nitrogen, sulfur, and chlorine. Examples for aliphatic compounds are methane (CH₄), ethylene and acetylene.

The term "unsaturated", as used herein, means that a moiety has one or more units of unsaturation, i.e. one or more double or triple bonds between carbon and/or heteroatoms.

The term "heteroaliphatic" refers to an aliphatic compound or an aliphatic group wherein at least one carbon atoms is replaced by a "heteroatom", i.e. a non-carbon atom, e.g. oxygen, sulfur, nitrogen or phosphorus. Heteroaliphatic groups may be substituted or unsubstituted, branched or unbranched, cyclic or acyclic.

The term "acyl" refers to groups formed by removing one or more hydroxy groups from an oxoacid, i.e. an acid containing oxygen, e.g. a carboxylic acid. The acyl group can have the structure RCO-, where R represents an alkyl group that is attached to the CO group with a single bond.

The term "alkyl" as used herein refers to branched or straight-chain (unbranched), saturated or unsaturated, aliphatic hydrocarbon groups, for example methyl, ethyl, n-propyl, i-propyl, n-butyl, t-butyl, i-butyl, pentyl, hexyl, heptyl, octyl, nonyl, decyl groups. The term comprises the term "cycloalkyl". The term "cycloalkyl" means a monocyclic saturated aliphatic hydrocarbon group. The term "cycloalkyl" includes, for example, cyclopropyl, methyl-cyclopropyl, 2,2-dimethyl-cyclobutyl, 2-ethyl-cyclopentyl and cyclohexyl.

The term "aryl" as used herein refers to monoyclic, bicyclic and polycyclic aromatic hydrocarbons, including a single ring or multiple aromatic rings fused or linked together where at least one part of the fused or linked rings forms the conjugated aromatic system. The aryl groups can typically have from 6 to 20 or more carbon atoms and can include, but are not limited to, e.g. phenyl, naphthyl, biphenyl, anthryl, tetrahydronaphthyl, phenanthryl, indene, benzonaphthyl, fluorenyl, and carbazolyl.

The term "heteroaryl", as used herein, represents a monocyclic, bicyclic or plycyclic ring, wherein at least one ring is aromatic and contains at least 1 heteroatom, e.g selected from the group consisting of O, N, P and S.

The term "halogen" refers to a group of elements comprising fluorine (F), chlorine (Cl), bromine (Br), iodine (I), and astatine (At).

The term "alkoxy" refers to the group R-O wherein R is alkyl. The term "aryloxy" refers to the group R-O wherein R is aryl. The term "heteroaryloxy" refers to a moiety having at least one aromatic ring that contains at least one ring heteroatom and an oxygen radical bonded to the aromatic ring, such as 4-pyrindinoxy. The term "alkylthio" refers to a linear or branched, saturated or unsaturated, non-aromatic hydrocarbon moiety containing a sulfur radical, such as -SCH₃ or -SCH=C₂H5. The term "arylthio" refers to a moiety having at least one aromatic ring and a sulfur radical bonded to the aromatic ring, such as phenylthio. The term "amino" refers to a molecule or moiety comprising the amino group -NH₂. The term "alkylamino" refers to a moiety having a nitrogen radical bonded to an alkyl group, such as -NHCH₃ or -NHCH=C₂H₅. The term "dialkylamino" refers to a moiety having a nitrogen radical bonded to two alkyl groups, such as -N(CH₃)₂ or -N(CH₃)(CH=C₂H₅). The term "arylamino" refers to a moiety having a nitrogen radical bonded to an aryl group, such as phenylamino. The term "diarylamino" refers to a moiety having a nitrogen radical bonded to two aryl groups, such as diphenylamino. The term "heteroarylthio" as used herein refers to a "heteroaryl" group linked through a divalent sulfur atom having its free valence bond from the sulfur atom. Representative examples include, but are not limited to furylthio, thienylthio, pyrrolylthio, imidazolylthio, pyrazolylthio, triazolylthio, pyridylthio, pyrazinylthio, pyrimidinylthio, pyridazinylthio, isothiazolylthio, isoxazolylthio, oxazolylthio, oxadiazolylthio, thiadiazolylthio, quinolylthio, isoquinolylthio, quinazolinylthio, quinoxalinnylthio, isoindolylthio, indolylthio, benzimidazolylthio, benzoxazolylthio, benzothiazolylthio, benzofuranylthio, tetrazolylthio, carbazolylthio, benzothienylthio, pteridinylthio,purinylthio and the like.

In a further aspect the present invention provides a method for making a compound(s) of the invention, wherein R¹ is a hydroxamic acid group CONHOH, generally according to scheme (I) below:

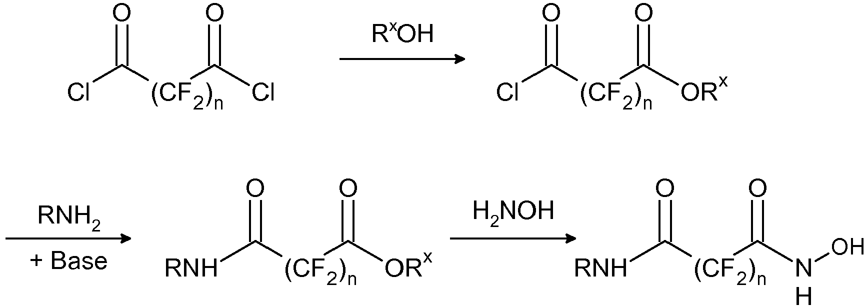

The base may be an inorganic or organic base like sodium carbonate, triethylamine or pyridine. H₂NOH is hydroxylamine, e.g. in the form of its inorganic salt or its aqueous solution in THF or any miscible solvent.

The method of the invention comprises the following steps:
(1) Reacting a perfluorinated compound (III) 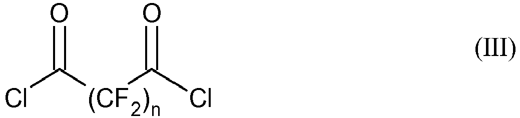 with a primary, secondary or tertiary alcohol R^{x}OH, wherein R^{x} is a cyclic or acyclic, substituted or unsubstituted, branched or unbranched aliphatic or aromatic,
(2) reacting the product (IV) of step (1) 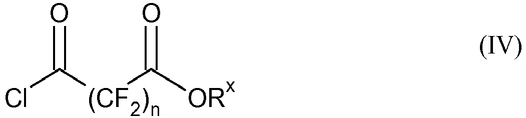 with RNH₂, wherein R is as defined above, and
(3) reacting the product (V) of step (2) 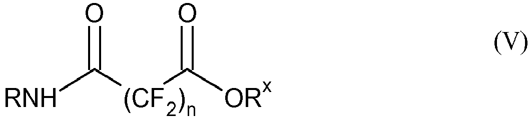 with H₂NOH.

In still a further aspect the present invention relates to pharmaceutical composition comprising a compound of the invention and a pharmaceutically acceptable carrier.

As used herein, the term "pharmaceutically acceptable carrier" encompasses any of the standard pharmaceutical carriers, and includes pharmaceutically acceptable excipients, diluents, fillers, disintegrants, lubricants and other agents that can function as a carrier. The term "pharmaceutically acceptable carrier" means a carrier that is useful in preparing a pharmaceutical composition that is generally safe and non-toxic, and includes carriers that are acceptable for veterinary use as well as for human pharmaceutical use. Such carriers can be solid, liquid, semisolid, or, in the case of an aerosol composition, gaseous. Pharmaceutical compositions are prepared in accordance with acceptable pharmaceutical procedures, such as described in Remingtons Pharmaceutical Sciences, 17th edition, ed. Alfonoso R. Gennaro, Mack Publishing Company, Easton, Pa. (1985). Pharmaceutically acceptable carriers are those that are compatible with the other ingredients in the formulation and biologically acceptable. The pharmaceutical composition may be constituted into any form suitable for the mode of administration selected. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

In further aspects the present invention relates to the compounds of the present invention for use as a medicament, in particular for use as a medicament for treating cancer.

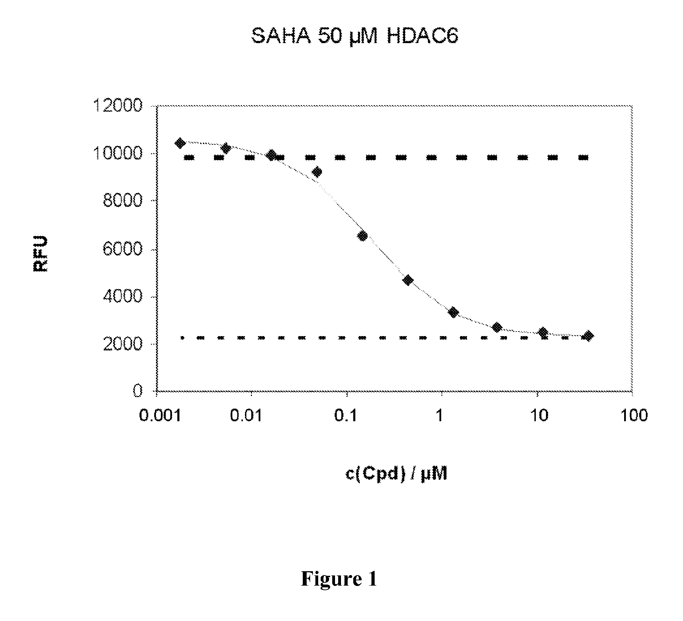

The present invention is further illustrated by way of non-limiting examples and figure 1.

Figure 1 shows a four parameter fit of a HDAC6/SAHA (compound) data set. Legend: Points = measure points; line = four parameter fit; dashed lines = controls. RFU = relative fluorescent units, c(Cpd) = concentration of compound (in µM = µmol/l).

### Examples

### Synthesis of example compounds

### 2,2,3,3,4,4,5,5,6,6,7,7-Dodecafluorooctanedioyldichloride ^{[13]}

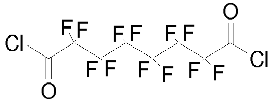

2,2,3,3,4,4,5,5,6,6,7,7-Dodecafluorooctanedioic acid (25.00 g, 64.09 mmol) was poured into a a flame dried three necked flask cooled under a nitrogen atmosphere. Thionylchloride (28.0 ml, 45.67 g, 383.9 mmol) was added via a dropping funnel and absol. DMF (8 drops) was added carefully to the colourless suspension. The reaction mixture was refluxed for 4 hours. After completion of the reaction remaining Thionylchloride was distilled off. The product was obtained as colourless oil by subsequent distillation of the residue under reduced pressure (b.p. 93-96 °C/100 mbar).
Yield: 23.46 g (54.95 mmol); 86 %
C₈Cl₂F₁₂O₂; M = 426.97 g/mol
¹⁹F-NMR (CDCl₃):
δ (ppm) = -117.6 - (-117.8) [m, 4F]; -125.8 - (-126.0) [m, 8F]

### Benzyl 7-chlorocarbonyl-2,2,3,3,4,4,5,5,6,6,7,7-dodecafluoroheptanoat

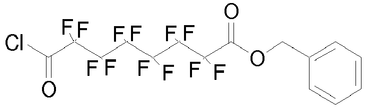

### Procedure 1:

2,2,3,3,4,4,5,5,6,6,7,7-Dodecafluorooctanedioyldichloride (23.46 g, 54.95 mmol) was poured into a flame dried three necked flask cooled under a nitrogen atmosphere. The starting material was cooled in an ice bath, before Benzyl alcohol (3.40 ml, 3.55 g, 32.83 mmol) was added via a dropping funnel under vigorous stirring over a period of one hour. The ice bath was removed and the reaction mixture was heated at 100 °C for one additional hour. After cooling to room temperature excessive starting material was collected by distillation under reduced pressure (b.p. 93 - 96 °C/100 mbar). High vacuum distillation of the residue provided the product as colourless oil (b.p. 90 - 92°C/0.1 mbar).
Yield: 7.70 g (15.44 mmol); 47 %

### Procedure 2:

2,2,3,3,4,4,5,5,6,6,7,7-Dodecafluorooctanedioyldichloride (18.16 g, 42.53 mmol) was dissolved in 50 ml absol. Dioxan and poured into a flame dried three necked flask cooled under a nitrogen atmosphere. The colourless solution was heated in an oil bath at 50 °C. A solution of Benzyl alcohol (3.10 ml, 3.24 g, 29.96 mmol) in 25 ml absol. Dioxan was added dropwise over a period of 90 minutes. The reaction mixture was stirred at 100 °C for additional 20 hours. After cooling to room temperature the solvent was removed by distillation in vacuo. High vacuum distillation of the residue provided the product as colourless oil (b.p. 90 - 92°C/0.1 mbar).
Yield: 6.83 g (13.70 mmol); 46 %
C₁₅H₇ClF₁₂O₃; M = 498.65 g/mol
¹H-NMR (CDCl₃):
δ (ppm) = 5.27 [s, 2H]; 7.24 - 7.41 [m, 5H]
¹⁹F-NMR (CDCl₃):
δ (ppm) = -113.9 - (-114.1) [m, 2F]; -119.4 - (-119.6) [m, 2F]; -122.1 - (-122.5) [m,4F]; - 122.6 - (-122.9) [m, 2F]; -123.5 - (-123.8) [m, 2F]

### Standard procedure 1:

### Step 1:

Benzyl 7-chlorocarbonyl-2,2,3,3,4,4,5,5,6,6,7,7-dodecafluoroheptanoat (0.50 g, 1.00 mmol) was dissolved in 10 ml absol. THF and poured into a flame dried three necked flask cooled under a nitrogen atmosphere. The colourless solution was cooled in an ice bath. Within 30 minutes a solution of Triethylamine (0.17 ml, 0.12 g, 1.19 mmol) and the particular aromatic amine or heteroaromatic amine (1.00 mmol) respectively in the required amount of absol. THF (10 - 20 ml) was added via a dropping funnel, maintaining the reaction temperature below 5 °C. The reaction mixture was stirred for two hours, before the ice bath was removed. After additional stirring for three hours the solvent was evaporated and the intermediate was purified by column chromatography.

### Step 2:

A solution of the particular Benzyl 7-arylcarbamoyl-2,2,3,3,4,4,5,5,6,6,7,7-dodecafluoro-heptanoat or Benzyl 7-heteroarylcarbamoyl-2,2,3,3,4,4,5,5,6,6,7,7-dodecafluoroheptanoat respectively in 10 ml absol. THF (cᵣₑₛ = 0.043 mmol/ml) was treated via syringe with the required amount of 1.0-M-Hydroxylamine (2.0 equivalents) in absol. THF. The reaction mixture was stirred at room temperature for 24 hours. After evaporation of the solvent the product was isolated by column chromatography.

### Standard procedure 2:

Benzyl 7-chlorocarbonyl-2,2,3,3,4,4,5,5,6,6,7,7-dodecafluoroheptanoat (0.50 g, 1.00 mmol) was dissolved in 10 ml absol. THF and poured into a flame dried two necked flask cooled under a nitrogen atmosphere. The colourless solution was cooled in an ice bath. Within 30 minutes a solution of Triethylamine (0.17 ml, 0.12 g, 1.19 mmol) and the particular aromatic amine or heteroaromatic amine (1.00 mmol) respectively in the required amount of absol. THF (10 - 20 ml) was added via a dropping funnel, maintaining the reaction temperature below 5 °C. The reaction mixture was stirred for two hours. After additional stirring for three hours at room temperature 1.0-M-Hydroxylamine (2.00 ml, 2.00 mmol) in absol. THF was added via syringe. The reaction mixture was stirred at room temperature until tlc indicated complete consumption of the intermediate. The solvent was evaporated and the product was isolated by column chromatography.

### Example 1: 2,2,3,3,4,4,5,5,6,6,7,7-Dodecafluorooctanedioic acid hydroxyamide phenyl-amide LU210

Using Aniline (0.10 ml; 0.10 g; 1.07 mmol) as starting material the product was obtained as a colourless solid according to standard procedure 2.
Yield: 0.07 g (0.15 mmol); 14 % 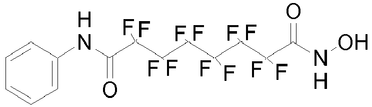 C₁₄H₈F₁₂N₂O₃; M = 480.21 g/mol
¹H-NMR (D₆-DMSO): δ (ppm) = 7.28 - 7.38 (m, 1H); 7.46 - 7.53 (m, 2H); 7.71 - 7.81 (m, 2H); 9.97 (s, br, 1H); 11.36 (s, br, 1H); 12.41 (s, br, 1H)

¹⁹F-NMR (D₆-DMSO):
δ (ppm) = -117.5 - (-117.8) [m, 2F]; -118.1 - (-118.5) [m, 2F]; -120.7 - (-121.3) [m, 4F]; - 121.5 - (121.7) [m, 2F]; -121.8 - (-122.0) [m, 2F]
MS (-p APCI): m/z = 479 (100 %) [M-1]⁻

### Example 2: 2,2,3,3,4,4,5,5,6,6,7,7-Dodecafluorooctanedioic acid hydroxyamide 4-methyl-phenylamide LU222

Using 4-Methylaniline (0.11 ml; 0.11 g; 1.03 mmol) as starting material the product was obtained as a yellowish solid according to standard procedure 2.
Yield: 0.09 g (0.18 mmol); 17 % 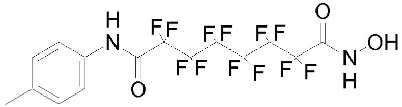 C₁₅H₁₀F₁₂N₂O₃; M = 494.23 g/mol
¹H-NMR (D₆-DMSO): δ (ppm) = 2.34 (s, 3H); 7.21 - 7.29 (m, 2H); 7.52 - 7.60 (m, 2H); 9.97 (s, br, 1H); 11.19 (s, br, 1H); 12.36 (s, br, 1H)
¹⁹F-NMR (D₆-DMSO):
δ (ppm) = -118.1 - (-118.3) [m, 2F]; -119.4 - (-119.7) [m, 2F]; -121.5 - (-121.9) [m, 4F];-122.2 - (-122.7) [m, 4F]
MS (-p APCI): m/z = 493 (100 %) [M-1]⁻

### Example 3: 2,2,3,3,4,4,5,5,6,6,7,7-Dodecafluorooctanedioic acid (4-chlorophenyl)amide hydroxyamide LU223

Using 4-Chloroaniline (0.13 g; 1.02 mmol) as starting material the product was obtained as a colourless solid according to standard procedure 2.
Yield: 0.07 g (0.14 mmol); 14 % 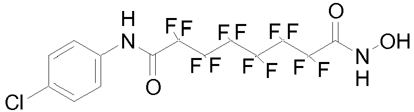 C₁₄H₇ClF₁₂N₂O₃; M = 514.65 g/mol
¹H-NMR (D₆-DMSO):
δ (ppm) = 7.48 - 7.53 (m, 2H); 7.69 - 7.78 (m, 2H); 9.93 (s, br, 1H); 11.41 (s, br, 1H); 12.37 (s, br, 1H)
¹⁹F-NMR (D₆-DMSO):
δ (ppm) = -119.5 - (-119.7) [m ,2F]; -119.8 - (-120.2) [m, 2F]; -121.7 - (-122.0) [m, 4F]; - 122.3 - (-123.1) [m, 4F]
MS (-p APCI): m/z = 515 (33 %) [M-1]⁻; 513 (100 %) [M-1]⁻

### Example 4: 2,2,3,3,4,4,5,5,6,6,7,7-Dodecafluorooctanedioic acid (4-acetylaminophenyl)-amide hydroxyamide LU224

Using 4-Acetylaminoaniline (0.15 g; 1.00 mmol) as starting material the product was obtained as a yellowish solid according to standard procedure 2.
Yield: 0.07 g (0.11 mmol); 11 % 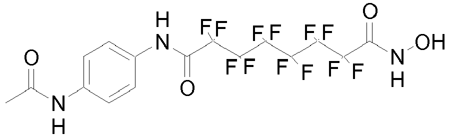 C₁₆H₁₁F₁₂N₃O₄; M = 537.26 g/mol
¹H-NMR (D₆-DMSO):
δ (ppm) = 2.18 (s, 3H); 7.60 - 7.82 (m, 4H); 10.03 (s, br, 1H); 10.13 (s, 1H); 11.27 (s, br, 1H); 12.44 (s, br, 1H)
¹⁹F-NMR (D₆-DMSO):

δ (ppm) =-118.7-(-119.1) [m, 2F]; - 119.5 - (-119.9) [m, 2F] ; 122.2 - (-123.1) [m, 8F]
MS (-p APCI): m/z = 536 (100 %) [M-1]⁻

### Example 5: 2,2,3,3,4,4,5,5,6,6,7,7-Dodecafluorooctanedioic acid (4-cyanophenyl)amide hydroxyamide LU225A

Using 4-Aminobenzonitrile (0.12 g; 1.02 mmol) as starting material the intermediate Benzyl 7-(4-cyanophenylcarbamoyl)-2,2,3,3,4,4,5,5,6,6,7,7-dodecafluoroheptanoat was obtained as a colouress solid according to standard procedure 1 step 1.
Yield: 0.15 g (0.26 mmol); 26 % 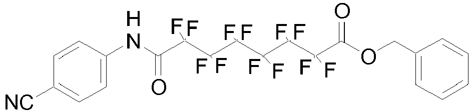 C₂₂H₁₂F₁₂N₂O₃; M = 580.07 g/mol
¹H-NMR (D₆-DMSO): δ (ppm) = 5.58 [s, 2H]; 7.45 - 7.55 [m, 5H]; 7.95 - 8.03 [m, 4H]; 11.72 [s, br, 1H]
¹⁹F-NMR (D₆-DMSO):

δ (ppm) = -118.1 - (-118.4) [m, 4F]; -121.3 - (-121.7) [m, 4F]; -122.0 - (-122.2) [m, 2F]; 122.4 - (-122.7) [m, 2F]
MS (-p APCI): m/z = 579 (100 %) [M-1]⁻

Using Benzyl 7-(4-cyanophenylcarbamoyl)-2,2,3,3,4,4,5,5,6,6,7,7-dodecafluoroheptanoat (0.13 g; 0.22 mmol) as starting material the product was obtained as a colourless solid according to standard procedure 1 step 2.
Yield: 0.04 g (0.08 mmol); 36 % 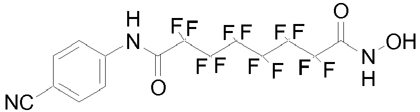 C₁₅H₇F₁₂N₃O₃; M = 505.22 g/mol
¹H-NMR (D₆-DMSO):
δ (ppm) = 7.96 - 8.01 (m, 4H); 10.00 (s, br, 1H); 11.73 (s, br, 1H); 12.41 (s, br, 1H)
¹⁹F-NMR (D₆-DMSO):
δ (ppm) = -118.5 - (-118.9) [m, 2F]; -119.2 - (-119.6) [m, 2F]; -121.5 - (-122.4) [m, 8F]
MS (-p APCI): m/z = 504 (100 %) [M-1]⁻

### Example 6: 2,2,3,3,4,4,5,5,6,6,7,7-Dodecafluorooctanedioic acid hydroxyamide (4-iodo-phenyl)amide LU227

Using 4-Iodoaniline (0.22 g; 1.00 mmol) as starting material the product was obtained as a colourless solid according to standard procedure 2.
Yield: 0.04 g (0.08 mmol); 8 % 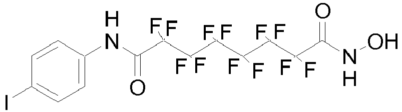 C₁₄H₇F₁₂IN₂O₃; M = 606.10 g/mol
¹H-NMR (D₆-DMSO):
δ (ppm) = 7.52 - 7.71 (m, 2H); 7.81 - 7.89 (m, 2H); 10.01 (s, br, 1H); 11.41 (s, br, 1H); 12.43 (s, br, 1H)
¹⁹F-NMR (D₆-DMSO):
δ (ppm) = -118.5 - (-118.8) [m, 2F]; -119.3 - (-119.7) [m, 2F]; -121.4 - (-121.9) [m, 4F];-122.0 - (-122.5) [m, 4F]
MS (-p APCI): m/z = 605 (100 %) [M-1]⁻

### Example 7: 2,2,3,3,4,4,5,5,6,6,7,7-Dodecafluorooctanedioic acid (3,4-dimethylphenyl)-amide hydroxyamide LU228

Using 3,4-Dimethylaniline (0.17 ml; 0.12 g; 0.99 mmol) as starting material the product was obtained as a colourless solid according to standard procedure 2.
Yield: 0.04 g (0.08 mmol); 8 % 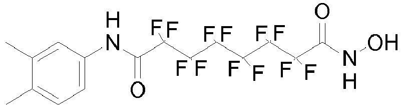 C₁₆H₁₂F₁₂N₂O₃; M = 508.26 g/mol ¹H-NMR (D₆-DMSO):
δ (ppm) = 2.19 - 2.28 (m, 6H); 7.11 - 7.20 (m, 1H); 7.33 - 7.40 (m, 1H); 7.42 - 7.48 (m, 1H); 9.92 (s, br, 1H); 11.11 (s, br, 1H); 12.35 (s, br, 1H)
¹⁹F-NMR (D₆-DMSO):
δ (ppm) = -118.2 - (-118.5) [m, 2F]; -119.1 - (-119.5) [m, 2F]; -121.5 - (-121.8) [m, 4F];-122.0 - (122.5) [m, 4F]
MS (-p APCI): m/z = 507 (100 %) [M-1]⁻

### Example 8: 2,2,3,3,4,4,5,5,6,6,7,7-Dodecafluorooctanedioic acid biphenyl-4-ylamide hydroxyamide LU229

Using Biphenyl-4-ylamine (0.17 g; 1.00 mmol) as starting material the product was obtained as a colourless solid according to standard procedure 2.
Yield: 0.08 g (0.14 mmol); 14 % 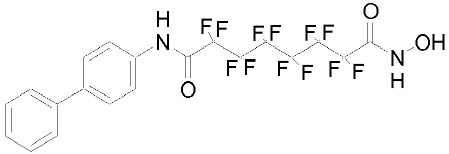 C₂₀H₁₂F₁₂N₂O₃; M = 556.30 g/mol ¹H-NMR (D₆-DMSO):
δ (ppm) = 7.31 - 7.39 (m, 1H); 7.39 - 7.53 (m, 2H); 7.67 - 7.82 (m, 6H); 9.97 (s, br, 1H); 11.36 (s, br, 1H); 12.36 (s, br, 1H)
¹⁹F-NMR (D₆-DMSO):
δ (ppm) = 116.9 - (-117.1) [m, 2F]; -117.7 - (-118.0); [m, 2F]; -120.3 - (-120.6) [m, 4F]; - 121.0 - (-121.2) [m, 2F]; -121.3 - (-121.5) [m, 2F]
MS (-p APCI): m/z = 555 (100 %) [M-1]⁻

### Example 9: 2,2,3,3,4,4,5,5,6,6,7,7-Dodecafluorooctanedioic acid (3-ethylphenyl)amide hydroxyamide LU232

Using 3-Ethylaniline (0.12 ml; 0.12 g; 1.00 mmol) as starting material the product was obtained as a colourless solid according to standard procedure 2.
Yield: 0.13 g (0.26 mmol); 26 % 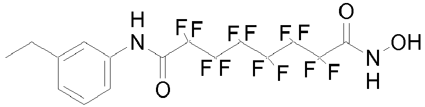 C₁₆H₁₂F₁₂N₂O₃; M = 508.26 g/mol
¹H-NMR (D₆-DMSO):
δ (ppm) = 1.17 - 1.21 (t, 3H, ³J = 7.0 Hz); 2.57 - 2.68 (q, 2H, ³J = 7.0 Hz); 7.07 - 7.13 (m, 1H); 7.29 - 7.37 (m, 1H); 7.47 - 7.55 (m, 1H); 9.95 (s, br, 1H); 11.19 (s, br, 1H); 12.35 (s, br, 1H)
¹⁹F-NMR (D₆-DMSO):
δ (ppm) = -116.9 - (-117.2) [m, 2F]; -117.6 - (-117.9) [m, 2F]; -120.3 - (-120.6) [m, 4F]; - 121.0 - (-121.3) [m, 2F]; -121.3 - (-121.6) [m, 2F]
MS (-p APCI): m/z = 507 (100 %) [M-1]⁻

### Example 10: 2,2,3,3,4,4,5,5,6,6,7,7-Dodecafluorooctanedioic acid hydroxyamide {4-[1-phenyl-1H-(1,2,3)triazol-4-yl]phenyl}amide LU233

Using 4-(4-Aminophenyl)-l-phenyl-1H-[1,2,3]-triazole (0.24 g; 1.02 mmol) as starting material the product was obtained as a colourless solid according to standard procedure 2.
Yield: 0.23 g (0.37 mmol); 36 % 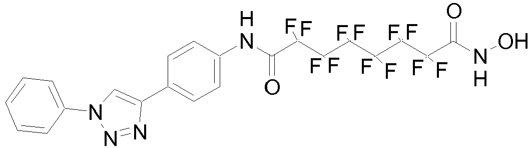 C₂₂H₁₃F₁₂N₅O₃; M = 623.35 g/mol
¹H-NMR (D₆-DMSO):
δ (ppm) = 7.39 - 7.56 [m, 2H]; 7.59 - 7.71 [m, 2H]; 7.78 - 7.88 [m, 2H]; 7.92 - 8.02 [m, 5H]; 9.32 [s, 1H]; 11.38 [s, br, 1H]
¹⁹F-NMR (D₆-DMSO):
δ (ppm) = -113.9 - (-114.3) [m, 2F]; -117.2 - (-117.6) [m, 2F]; -120.5 - (-121.5) [m, 8F]
MS (-p APCI): m/z = 622 (4 %) [M-1]⁻; 607 (100 %) [M-15]⁻; 563 (100 %) [M-59]⁻
4-(4-Aminophenyl)-1-phenyl-1H-[1,2,3]-triazole 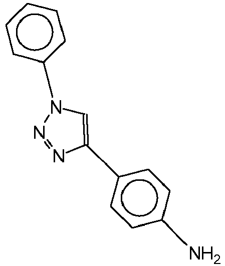 was prepared this way:

A 100 mL 2 necked flask was charged with a mixture of 25mL t-butanol and 25 mL water. Then were added 2.40 g (20.00 mmol) phenylazide^{[14]} and 2.36 g (20.00 mmol; 1 eq) 4-aminophenylethyne. Afterwards were given 2 mL of a 1 m sodium ascorbate solution (2.00 mmol; 0.1 eq.) and 50 mg of copper-II-sulfate pentahydrate (0.20 mmol; 0.01 eq.). The dark green suspension was then heated at 60° C for 17 hours. 50 mL of water were added to the reaction mixture after cooling down to room temperature. The mixture was then filtered and washed with water, giving a beige yellow solid,
3.50 g (14.75 mmol); 74%
C₁₄H₁₃N₄; M = 237.28 g/mol
¹H-NMR (DMSO):
δ (ppm) = 5.42 (s, br, 2H); 6.76 (d, 2H); 7.41 (m, 1H); 7.52 (m, 4H); 7.95 (d, 2H); 9.05 (s, 1H)
¹³ C-NMR (DMSO):
δ (ppm) = 113.94; 117.11; 117.73; 119.74; 126.40; 128.35; 129.83; 136.78; 148.33; 148.95
R_{F} (diethylether): 0.54

### Example 11: 2,2,3,3,4,4,5,5,6,6,7,7-Dodecafluorooctanedioic acid hydroxyamide pyren-1-ylamide LU234

Using 1-Aminopyren (0.22 g; 1.01 mmol) as starting material the product was obtained as a brownish solid according to standard procedure 2.
Yield: 0.14 g (0.23 mmol); 23 % 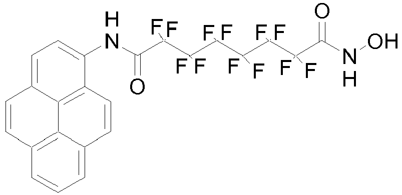 C₂₄H₁₂F₁₂N₂O₃; M = 604.34 g/mol
¹H-NMR (D₆-DMSO):
δ (ppm) = 8.03 - 8.44 [m, 9H]; 9.99 [s, br, 1H]; 11.94 [ s, br, 1H]; 12.43 [s, br, 1H]
¹⁹F-NMR (D₆-DMSO):
δ (ppm) = -117.2 - (-117.5) [m, 2F]; -118.0 - (-118.3) [m, 2F]; -120.5 - (-121.1) [m, 4F]; - 121.3 - (-121.6) [m, 2F]; -121.8 - (-122.1) [m, 2F]
MS (-p APCI): m/z = 603 (100 %) [M-1]⁻

### Example 12: 2,2,3,3,4,4,5,5,6,6,7,7-Dodecafluorooctanedioic acid hydroxyamide (4-phenylthiazol-2-yl)amide LU235

Using 2-Amino-4-phenylthiazole (0.18 g; 1.02 mmol) as starting material the product was obtained as a brownish solid according to standard procedure 2.
Yield: 0.07 g (0.12 mmol); 12 % 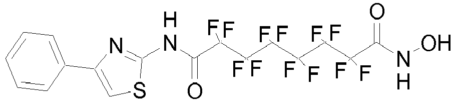 C₁₇H₉F₁₂N₃O₃S; M = 563.32 g/mol
¹H-NMR (D₆-DMSO):
δ (ppm) = 7.37 - 7.55 [m, 4H]; 7.72 [s, 1H]; 7.85 - 7.92 [m, 1H]; 9.94 [s, br, 1H]; 12.33 [s, br, 1H]; 14.45 [s, br, 1H]
¹⁹F-NMR (D₆-DMSO):
δ (ppm) = -116.3 - (-116.8) [m, 2F]; -118.2 - (-118.5) [m, 2F]; -120.7 - (-121.3) [m, 4F]; - 121.3 - (-121.6) [m, 2F]; -121.8 - (-122.1) [m, 2F]
MS (-p APCI): m/z = 562 (100 %) [M-1]⁻

### Example 13: 2,2,3,3,4,4,5,5,6,6,7,7-Dodecafluorooctanedioic acid hydroxyamide [4-(1-phenyl-1H-[1,2,3]triazol-5-yl)phenyl]amide LU236

Using 5-(4-Aminophenyl)-1-phenyl-1H-[1,2,3]-triazole (0.24 g; 1.02 mmol) as starting material the product was obtained as a brownish solid according to standard procedure 2.
Yield: 0.18 g (0.29 mmol); 28 % 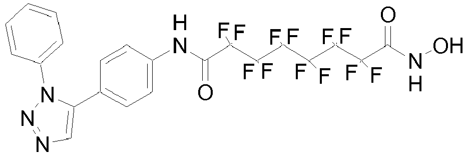 C₂₂H₁₃F₁₂N₅O₃; M = 623.35 g/mol
¹H-NMR (D₆-DMSO):
δ (ppm) = 7.32 - 7.38 [m, 2H]; 7.41 - 7.49 [m, 2H]; 7.54 - 7.61 [m, 3H]; 7.68 - 7.75 [m, 2H]; 8.17 [s, 1H]; 9.93 [s, br, 1H]; 11.38 [s, br, 1H]; 12.38 [s, br, 1H]
¹⁹F-NMR (D₆-DMSO):
δ (ppm) = -114.6 - (-114.9) [m, 2F]; -117.7 - (-118.0) [m, 2F]; -120.8 - -121.9 [m, 8F]
MS (-p APCI): m/z = 622 (9 %) [M-1]⁻; 607 (72 %) [M-15]⁻; 563 (100 %) [M-59]⁻
5-(4-Aminophenyl)-1-phenyl-1H-[1,2,3]-triazole 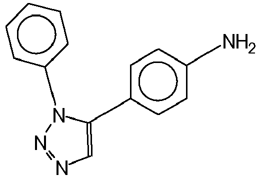 was prepared according to the following procedure:

A 100 mL 2 necked flask was charged with 50 mL of toluene and 2.36 g (20.00 mmol; 1 eq) 4-aminophenylethyne. 2.40 g (20.00 mmol) phenylazide were then added and the black solution was heated to reflux for 43 hours. The toluene was removed at reduced pressure and the residue was resolved in 2 n hydrochloric acid. Unsoluble material was separated and the liquid phase was washed three times with ethylacetate afterwards. The aqueous phase was then heated with some charcoal, filtrated and neutralised with conc. sodium hydroxide solution. The black material which deposited from the solution was filtrated, dried and crystallised from a mixture ofwater/ethanol. Brown crystals were then separated and dried.
1.2 g (14.75 mmol); 25 %
C₁₄H₁₃N₄; M = 237.28 g/mol
¹H-NMR (DMSO):
δ (ppm) = 5.39 (s, br, 2H); 6.56 (d, 2H); 6.85 (m, 2H); 7.41 (m, 2H); 7.56 (m, 2H); 7.95 (s, 1H)
¹³C-NMR (DMSO):
δ (ppm) = 112.44; 113.59; 127.17; 128.58; 128.75; 129.42; 130.52; 131.63; 138.42; 149.66
R_{F} (diethylether): 0.58
MS (+p APCI): m/z = 237 (100 %) [MH⁺]

### Example 14: 2,2,3,3,4,4,5,5,6,6,7,7-Dodecafluorooctanedioic acid hydroxyamide naphthalen-1-ylamide LU239

Using Naphthalen-1-ylamine (0.14 g; 0.98 mmol) as starting material the product was obtained as a colourless solid according to standard procedure 2.
Yield: 0.06 g (0.11 mmol); 11 % 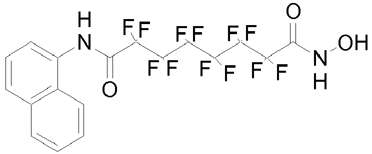 C₁₈H₁₀F₁₂N₂O₃; M = 530.26 g/mol
¹H-NMR (D₆-DMSO):
δ (ppm) = 7.56 - 7.59 (m, 1H); 7.64 - 7.73 (m, 3H); 7.83 - 7.91 (m, 1H); 8.04 - 8.14 (m, 2H); 10.02 (s, br, 1H); 10.62 (s, br, 1H); 12.44 (s, br, 1H)
¹⁹F-NMR (D₆-DMSO):
δ (ppm) = -117.3 - (-117.5) [m, 2F]; -118.1 - (-118.4) [m, 2F]; -121.4 - (-122.3) [m, 4F]; - 122.6 - (-123.1) [m, 2F]; -123.3 - (-123.8) [m, 2F]
MS (-p APCI): m/z = 529 (100 %) [M-1]⁻

### Example 15: 2,2,3,3,4,4,5,5,6,6,7,7-Dodecafluorooctanedioic acid biphenyl-3-ylamide hydroxyamide LU242

Using Biphenyl-3-ylamine (0.17 g; 1.00 mmol) as starting material the product was obtained as a beige coloured solid according to standard procedure 2.
Yield: 0.08 g (0.14 mmol); 14 % 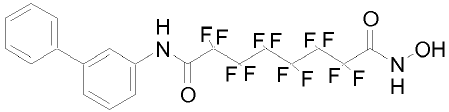 C₂₀H₁₂F₁₂N₂O₃; M = 556.30 g/mol
¹H-NMR (D₆-DMSO):
δ (ppm) = 7.39 - 7.43 [m, 1H]; 7.45 - 7.58 [m, 4H]; 7.63 - 7.73 [m, 3H]; 7.94 - 7.98 [m, 1H]; 9.94 [s, br, 1H]; 11.35 [s, br, 1H]; 12.37 [s, br, 1H]
¹⁹F-NMR (D₆-DMSO):
δ (ppm) = -116.9 - (-117.2) [m, 2F]; -117.6 - (-117.9) [m, 2F]; -120.0 - (-120.4) [m, 4F]; - 120.8 - (-121.1) [m, 2F]; 121.2 - (-121.5) [m, 2F]
MS (-p ESI): m/z = 555 (100 %) [M-1]⁻

### Example 16: 2,2,3,3,4,4,5,5,6,6,7,7-Dodecafluorooctanedioic acid hydroxyamide naphthalen-2-ylamide LU243

Using Naphthalen-2-ylamine (0.14 g; 0.98 mmol) as starting material the product was obtained as a beige coloured solid according to standard procedure 2.
Yield: 0.08 g (0.15 mmol); 15 % 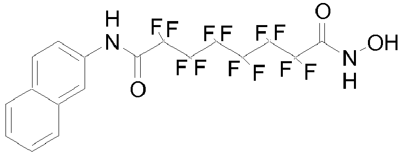 C₁₈H₁₀F₁₂N₂O₃; M = 530.26 g/mol
¹H-NMR (D₆-DMSO):
δ (ppm) = 7.55 - 7.64 [m, 2H]; 7.87 - 8.04 [m, 1H]; 7.91 - 8.05 [m, 3H]; 8.37 - 8.40 [m, 1H]; 10.02 [s, br, 1H]; 11.53 [s, br, 1H]; 12.42 [s, br, 1H]
¹⁹F-NMR (D₆-DMSO):
δ (ppm) = -116.8 - (-117.2) [m, 2F]; -117.6 - (-118.0) [m, 2F]; -120.2 - (-120.7) [m, 4F]; 121.0 - (-121.3) [m, 2F]; -121.4 - (-121.7) [m, 2F]
MS (-p ESI): m/z = 529 (100 %) [M-1]⁻

### Example 17: 2,2,3,3,4,4,5,5,6,6,7,7-Dodecafluorooctanedioic acid biphenyl-2-ylamide hydroxyamide LU244

Using Biphenyl-2-ylamine (0.17 g; 1.00 mmol) as starting material the product was obtained as a colourless solid according to standard procedure 2.
Yield: 0.07 g (0.13 mmol); 13 % 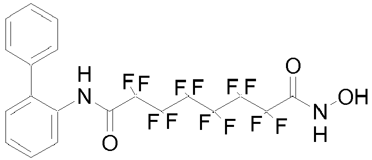 C₂₀H₁₂F₁₂N₂O₃; M = 556.30 g/mol
¹H-NMR (D₆-DMSO):

δ (ppm) = 7.31 - 7.53 [m, 9H]; 9.93 [s, br, 1H]; 11.05 [s, br, 1H]; 12.35 [s, br, 1H]
¹⁹F-NMR (D₆-DMSO):
δ (ppm) = -117.5 - (-117.8) [m, 2F]; -118.2 - (-118.5) [m, 2F]; -120.8 - (-121.3) [m, 4F]; - 121.6 - (-121.9) [m, 2F]; -121.9 - (-122.2) [m, 2F]
MS (-p ESI): m/z = 555 (100 %) [M-1]⁻

### Example 18: 2,2,3,3,4,4,5,5,6,6,7,7-Dodecafluorooctanedioic acid anthracen-2-ylamide hydroxyamide LU245

Using Anthracen-2-ylamine (0.20 g; 1.03 mmol) as starting material the product was obtained as a brownish solid according to standard procedure 2.
Yield: 0.08 g (0.14 mmol); 14 % 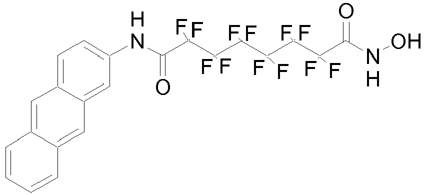 C₂₂H₁₂F₁₂N₂O₃; M = 580.32 g/mol
¹H-NMR (D₆-DMSO):
δ (ppm) = 7.48 - 7.57 [m, 2H]; 7.71 - 7.77 [m, 1H]; 8.05 - 8.28 [m, 3H]; 8.48 - 8.62 [m, 3H]; 9.94 [s, br, 1H]; 11.51 [s, br, 1H]; 12.38 [s, br, 1H]
¹⁹F-NMR (D₆-DMSO):
δ (ppm) = -117.3 - (-117.6) [m, 2F]; -118.1 - (-118.4) [m, 2F]; -120.8 - (-121.3) [m, 4F]; - 121.5 - (-121.7) [m, 2F]; -121.8 - (-122.1) [m, 2F]
MS (-p ESI): m/z = 579 (100 %) [M-1]⁻

### Example 19: 2,2,3,3,4,4,5,5,6,6,7,7-Dodecafluorooctanedioic acid hydroxyamide phenanthren-9-ylamide LU246

Using Phenanthren-9-ylamine (0.20 g; 1.03 mmol) as starting material the product was obtained as a yellowish solid according to standard procedure 2.
Yield: 0.03 g (0.05 mmol); 5 % 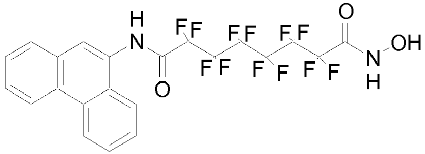 C₂₂H₁₂F₁₂N₂O₃; M = 580.32 g/mol
¹H-NMR (D₆-DMSO):
δ (ppm) = 7.69 - 7.98 [m, 6H]; 8.04 - 8.11 [m, 1H]; 8.89 - 8.99 [m, 2H]; 9.97 [s, br, 1H]; 11.63 [s, br, 1H]; 12.46 [s, br, 1H]
¹⁹F-NMR (D₆-DMSO):
δ (ppm) = -117.3 - (-117.6) [m, 2F]; -118.3 - (-118.6) [m, 2F]; -120.7 - (-121.2) [m, 4F]; - 121.3 - (-121.6) [m, 2F]; -121.8 - (-122.1) [m, 2F]
MS (-p ESI): m/z = 579 (100 %) [M-1]⁻

### Example 20: 2,2,3,3,4,4,5,5,6,6,7,7-Dodecafluorooctanedioic acid anthracen-1-ylamide hydroxyamide LU247

Using Anthracen-1-ylamine (0.20 g; 1.03 mmol) as starting material the product was obtained as a brownish solid according to standard procedure 2.
Yield: 0.10 g (0.17 mmol); 13 % 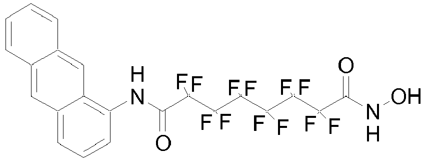 C₂₂H₁₂F₁₂N₂O₃; M = 580.32 g/mol
¹H-NMR (D₆-DMSO):
δ (ppm) = 7.55 - 7.80 [m, 4H]; 8.10 - 8.23 [m, 3H]; 8.49 [s, 1H]; 8.78 [s, 1H]; 10.03 [s, br, 1H]; 11.75 [s, br, 1H]; 12.48 [s, br, 1H]
¹⁹F-NMR (D₆-DMSO):
δ (ppm) = -117.3 - (-117.6) [m, 2F]; -118.3 - (-118.5) [m, 2F]; 120.7 - (-121.1) [m, 4F]; - 121.6 - (-121.8) [m, 2F]; -121.9 - (-122.2) [m, 2F]
MS (-p ESI): m/z = 579 (100 %) [M-1]⁻

### HDAC inhibition by compounds of the invention

In order to evaluate the inhibitory characteristics of example compounds of the invention, IC₅₀ values were determined for such compounds. The IC₅₀ value is the half maximal (50%) inhibitory concentration (IC) of a substance (50% IC, or IC50), i.e. the concentration of a given compound that is necessary to inhibit a given biological process, e.g. an enzyme activity, by half. A two-step assay was used for this purpose.

### Two-step assay for determination of IC₅₀

To obtain the IC₅₀ value for an individual compound/HDAC pair, a two-step assay can be performed based on a fluorescence signal. In principle, an ε-acetylated lysine substrate is deacetylated by histone-deacetylases in the first step. Trypsin releases detectable 7-amino-4-methylcoumarin (ex = excitation wavelength: 390 nm, em = emission wavelength: 460 nm) by cleaving the substrate at the deacetylated lysine in a subsequent reaction^{[9]}. The enzyme activity is inhibited by a suitable HDACi.

The obtained data are interpreted using a four parameter fit. Based on this fit, the IC₅₀ value can be estimated. Analysis ot the data and calculation of the IC50 values was accomplished using a four-parameter logistic model protocol as previously described by Vølund^{[16]}. For nonlinear fits the Levenberg-Marquardt algorithm was applied^{[17],[18]}. An example data set with HDAC6 and the known inhibitor SAHA, already processed with the four parameter fit, is presented in Figure 1. Results for IC₅₀ are presented in Table 1 below.

**Table 1: IC₅₀ values in µM for synthesized compounds of the invention with a perfluorinated linker. The compounds RK216 and LU197 were used as negative control, SAHA as positive control. HDAH = HDAC-like amidohydrolase**

| **Compound** | **Abbreviation** | **HDAC1** | **HDAC8** | **HDAC6** | **HDAC7** | **HDAH** |
|---|---|---|---|---|---|---|
| **RK216** | | **>100** | **>100** | **43.96** | **>100** | **34.76** |
| **LU197** | | **>100** | **>100** | **>100** | **>100** | **52.67** |
| **LU210** | **Ph/Perfluor-SAHA** | **17.91** | **28.82** | **12.48** | **14.76** | **1.67** |
| **LU222** | **4-Me-Ph** | **16.33** | **87.62** | **>100** | **84.78** | **0.86** |
| **LU223** | **4-Cl-Ph** | **15.06** | **72.98** | **26.30** | **26.77** | **0.72** |
| **LU224** | **4-CH3CONH-Ph** | **21.80** | **76.25** | **10.45** | **21.47** | **3.41** |
| **LU225 A** | **4-CN-Ph** | **21.83** | **73.94** | **>100** | **53.65** | **0.70** |
| **LU227** | **4-I-Ph** | **16.93** | **75.84** | **5.13** | **6.86** | **0.91** |
| **LU228** | **3,4-DiMe-Ph** | **10.03** | **22.75** | **7.80** | **5.51** | **0.66** |
| **LU229** | **4-Ph-Ph** | **37.89** | **>100** | **59.12** | **22.03** | **0.78** |
| **LU232** | **3-Et-Ph** | **19.92** | **21.29** | **1.55** | **4.97** | **9.18** |
| **LU 233** | **4-(1-Ph-Tri-4-yl)-Ph** | **>100** | **>100** | **9.81** | **42.17** | **16.92** |
| **LU234** | **Pyren-1-yl** | **28.30** | **77.84** | **20.24** | **>100** | **1.48** |
| **LU235** | **4-Ph-Thia-2-yl** | **3.31** | **2.10** | **6.47** | **1.77** | **5.11** |
| **LU236** | **4-(1-Ph-Tri-5-yl)-Ph** | **83.15** | **>100** | **56.57** | **16.35** | **10.38** |
| **SAHA** | **Virostat** | **0.04** | **3.95** | **0.21** | **>100** | **0.81** |

The compounds RK216 and LU197 were used to check the validity of the assay. Both compounds should show no activity due to their structural properties. The C6 alkyl chain in compound RK 216 is conformational bonded into a cyclohexane moiety. Compound LU197 has two benzylester groups at the endings, so the zinc-binding group is missing (see below for structures).

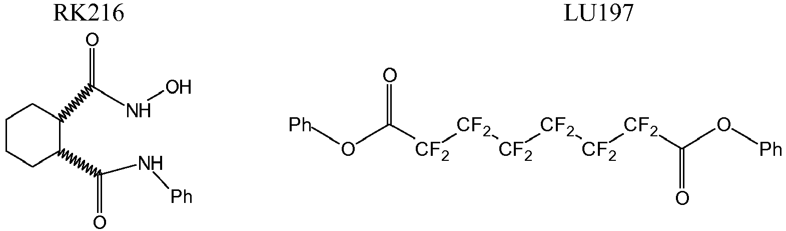

In Table 1 it is evident that both compounds, i.e. RK 216 and LU197, show no activity. Further results with the synthesized compounds of the invention confirmed the predicted inhibitory effect when applied to different human HDAC classes (HDAC1, HDAC6, HDAC7 and HDAC8). The bacterial isoform HDAH is inhibited in a noticeable range too. Through these results the inhibitory potential of compounds with perfluorinated linker are revealed. Additionally, strong signs of structure based HDAC isoform selectivity can be observed. In summary, the results demonstrate the successful synthesis of novel HDAC inhibitors exhibiting unusual HDAC isoform selectivities. Some of the compounds are clearly selective for class II HDACs, particularly HDAC6. Therefore, compounds with perfluorinated linker and improved isoform selectivity should be valuable research tools and maybe potential lead structures for the development of drugs for the treatment of cancer and other diseases linked to unregulated HDACs, particularly class II HDACs^{[10][11][12]}. Most of the side effects associated with the usage of approved cancer therapeutic SAHA, which inhibits both class I and class II HDACs (see table), are attributed to its effect against class I HDACs. Hence, class II selective HDAC inhibitors are supposed to cause less adverse effects.

### Citation List

[1] Boyle, P., Levin, B. (2008) "World Cancer Report", IARC Nonserial Publication.
[2] CA Cancer J Clin 2008; 58:71-96
[3] American Cancer Society. Cancer Facts & Figures 2009. Atlanta: American Cancer Society; 2009.
[4] Anton V. Bieliauskas; M. K. H. Pflum 2008 Isoform selective histone deacetylase inhibitors
[5] Finnin M. S., Donigian J. R., Cohen A., Richon V. M., Rifkind R. A., Marks P. A., Breslow R., Pavletich N. P, Nature 1999, 401, 188-193.
[6] David S. Schrump Cytotoxicity mediated by histone deacetylase inhibitors in cancer cells: Mechanisms and potential clinical implications. Clin Cancer Res; 2009, 15 (12), 3947-3957.
[7] P. A. Marks and W.-S.Xu, Histone deacetylase inhibitors: potential in cancer therapy. J. of Cellular biochemistry; 2009, 107, 600-608.
[8] NakagawaM., Oda Y., Eguchi T., et al. Expression profile of class I histone deacetylases in human cancer tissues. Oncol Rep, 2007, 18, 763-74
[9] D. Wegener et al.: Improved fluorogenic histone deacetylase assay for high-throughput-screening applications, Analytical Biochemistry, 2003, 321, 202-208.
[10] O. Witt and R. Lindemann. HDAC inhibitors: Magic bullets, dirty drugs or just another targeted therapy, Cancer Letters, 2009, 280, 123-124.
[11] O. Witt et al.: HDAC family: What are the cancer relevant targets? Cancer Letters 2009, 277, 8-21.
[12] O. Witt et al.: Targeting of HDAC8 and investigational inhibitors in neuroblastoma. Expert Opin. Investig. Drugs, 2009, 18 (11), 1605-1617.
[13] N. Steinhauser and R. Mühlhaupt, Polymer Bulletin, 1994, 32, 403-410.
[14] H. Perlinger et al., Chem. Ber., 1958, 91, 2330-6.
[15] Paris et al., J. Med. Chem. 2008, 51, 1505-1529
[16] Volund, A. (1978), Application of the four-parameter logistic model to bioassay: comparison with slop ration and parallel line models, Biometrics 34: 357-365
[17] Levenberg, K. (1944), A method for the solution of certain non-linear problems in least squares, The Quarterly of Applied Mathematics 2: 164-168.
[18] D.W. Marquardt, An algorithm for least-squares estimation of nonlinear parameters, Journal of the Society for Industrial and Applied Mathematics, 11(2):431-441, 1963.

## Claims

1. A histone deacetylase (HDAC) inhibiting compound of the formula (I) 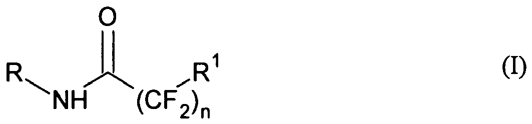 or a pharmaceutically acceptable salt thereof, wherein
n is 4, 5, 6, 7, 8, 9, 10, 11, 12, 13 or 14,
R is cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl, and
R¹ is a metal binding group capable of binding a metal ion within an active site of said histone deacetylase.

2. A compound according to claim 1, wherein n is 4, 5, 6, 7 or 8, preferably 5, 6 or 7, further preferred 6 or 7, and especially preferred 6.

3. A compound according to claim 1 or 2, wherein R¹ is one of the following

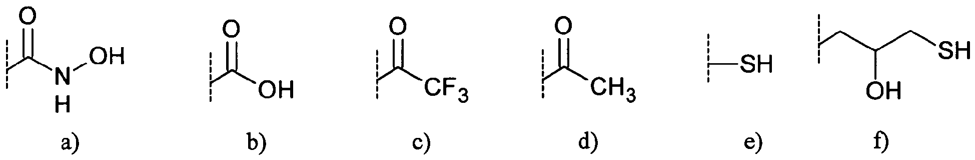

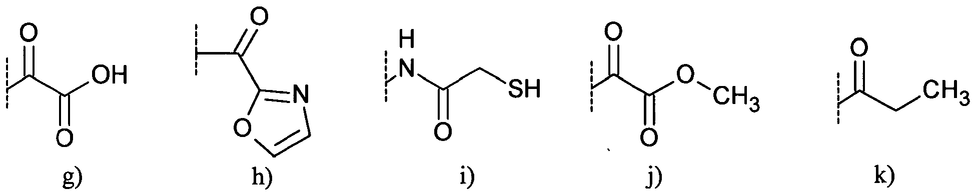

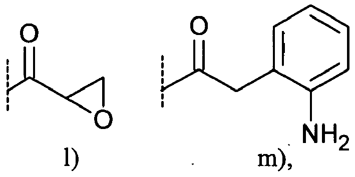

4. A compound according to one of the preceding claims, wherein
R is 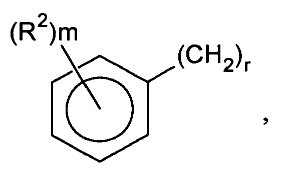 (R²)m (CH₂)r
wherein
m is 0, 1, 2, 3, 4 or 5
r is 0, 1, 2 or 3, and wherein
R² is hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl.

5. A compound according to claim 4, wherein R² is one of the following: 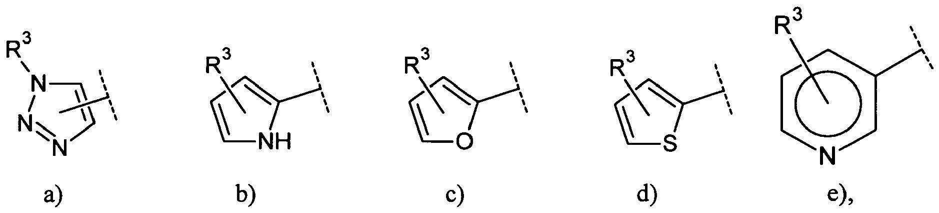 and wherein R³ is hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl.

6. A compound according to claim 1, wherein R is one of the following: 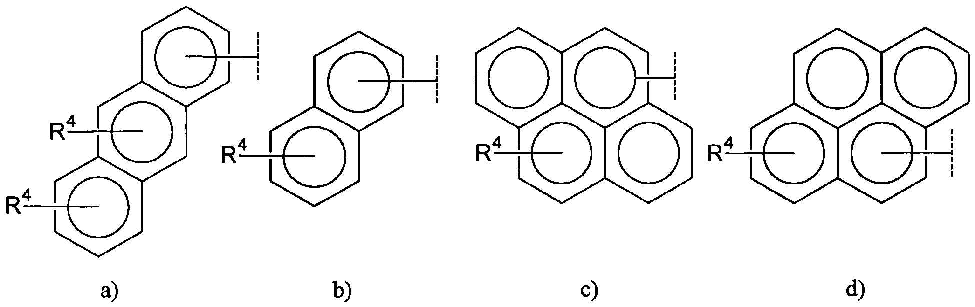 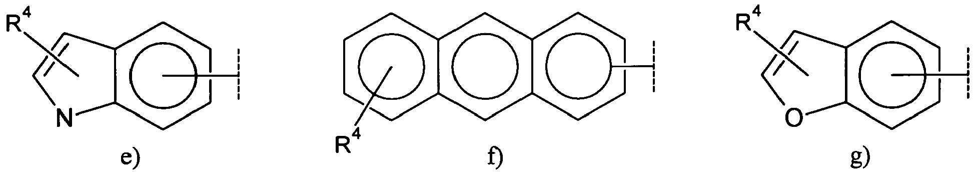 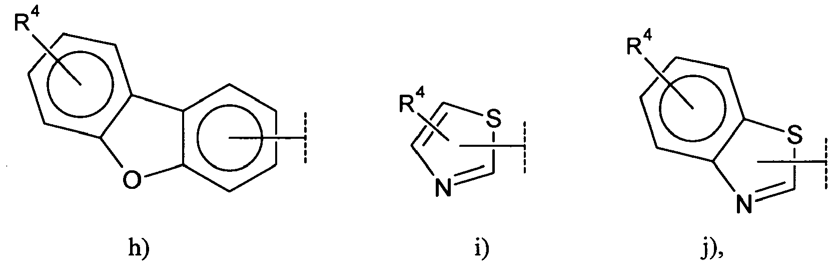 and wherein R⁴ is cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl.

7. Method for making a compound according to one of claims 1 to 6, R¹ being a hydroxamic acid group CONHOH, comprising the steps of
(1) Reacting a perfluorinated compound (III) 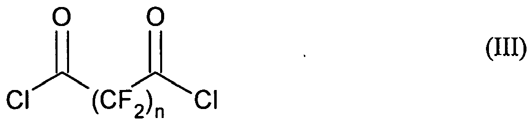 with a primary, secondary or tertiary alcohol RₓOH, wherein Rₓ is a cyclic or acyclic, substituted or unsubstituted, branched or unbranched aliphatic or aromatic,
(2) reacting the product (IV) of step (1) 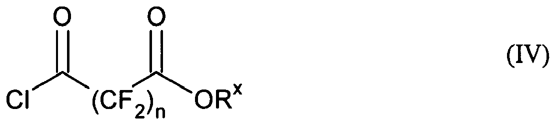 with RNH₂, and
(3) reacting the product (V) of step (2) 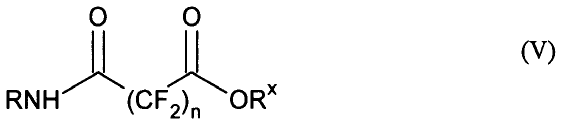 with H₂NOH.

8. A pharmaceutical composition comprising a compound of any one of claims 1 to 6 and a pharmaceutically acceptable carrier.

9. A compound of one of claims 1 to 6 for use as a medicament.

10. A compound of one of claims 1 to 6 for use as a medicament for treating cancer.

## Patentansprüche

1. Histon-Deacetylase (HDAC) hemmende Verbindung der Formel (I) 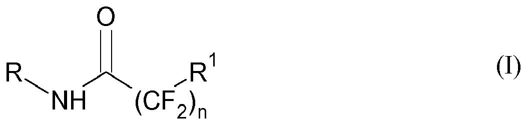 oder ein pharmazeutisch annehmbares Salz davon, wobei
n 4, 5, 6, 7, 8, 9, 10, 11, 12, 13 oder 14 ist,
R cyclischer oder acyclischer, substituierter oder unsubstituierter, verzweigter oder unverzweigter Aliphat; cyclischer oder acyclischer, substituierter oder unsubstituierter, verzweigter oder unverzweigter Heteroaliphat; substituiertes oder unsubstituiertes, verzweigtes oder unverzweigtes Acyl; substituiertes oder unsubstituiertes, verzweigtes oder unverzweigtes Aryl; substituiertes oder unsubstituiertes, verzweigtes oder unverzweigtes Heteroaryl ist, und R¹ eine metallbindende Gruppe ist, die in der Lage ist, ein Metallion innerhalb eines aktiven Zentrums der Histon-Deacetylase zu binden.

2. Verbindung nach Anspruch 1, wobei n 4, 5, 6, 7 oder 8, vorzugsweise 5, 6 oder 7, weiter bevorzugt 6 oder 7, und besonders bevorzugt 6 ist.

3. Verbindung nach Anspruch 1 oder 2, wobei R¹ eines von folgendem ist

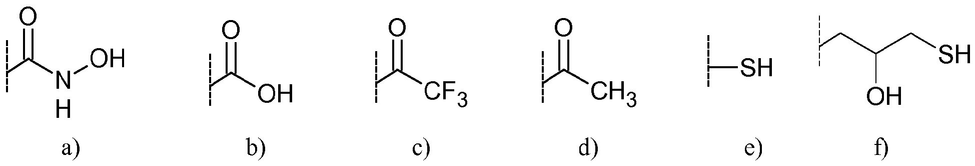

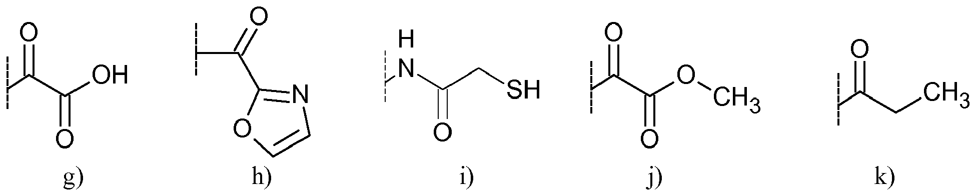

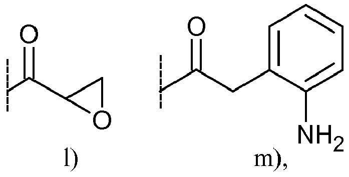

4. Verbindung nach einem der vorhergehenden Ansprüche, wobei
R= 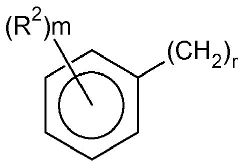 ist,
wobei
m 0, 1, 2, 3, 4 oder 5 ist
r 0, 1, 2 oder 3 ist, und wobei
R² Wasserstoff; Halogen; cyclischer oder acyclischer, substituierter oder unsubstituierter, verzweigter oder unverzweigter Aliphat; cyclischer oder acyclischer, substituierter oder unsubstituierter, verzweigter oder unverzweigter Heteroaliphat; substituiertes oder unsubstituiertes, verzweigtes oder unverzweigtes Acyl; substituiertes oder unsubstituiertes, verzweigtes oder unverzweigtes Aryl; substituiertes oder unsubstituiertes, verzweigtes oder unverzweigtes Heteroaryl ist.

5. Verbindung nach Anspruch 4, wobei R² eines von folgendem ist: 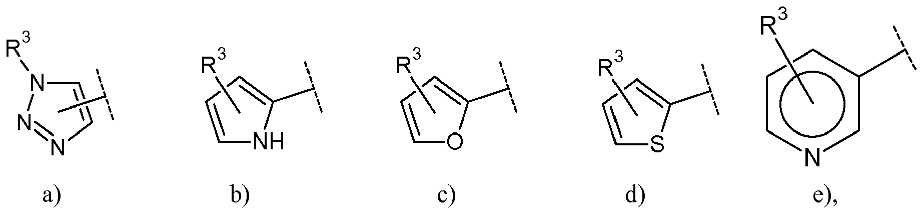 und wobei R³ Wasserstoff; Halogen; cyclischer oder acyclischer, substituierter oder unsubstituierter, verzweigter oder unverzweigter Aliphat; cyclischer oder acyclischer, substituierter oder unsubstituierter, verzweigter oder unverzweigter Heteroaliphat; substituiertes oder unsubstituiertes, verzweigtes oder unverzweigtes Acyl; substituiertes oder unsubstituiertes, verzweigtes oder unverzweigtes Aryl; substituiertes oder unsubstituiertes, verzweigtes oder unverzweigtes Heteroaryl ist.

6. Verbindung nach Anspruch 1, wobei R eines von folgendem ist: 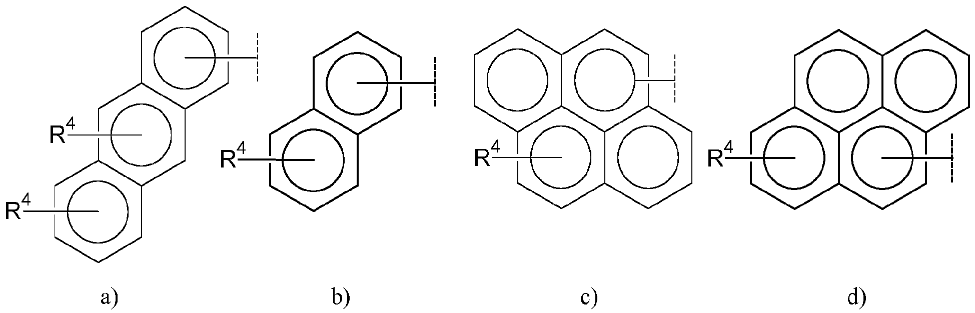 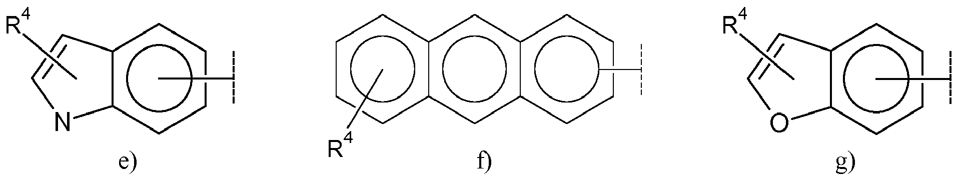 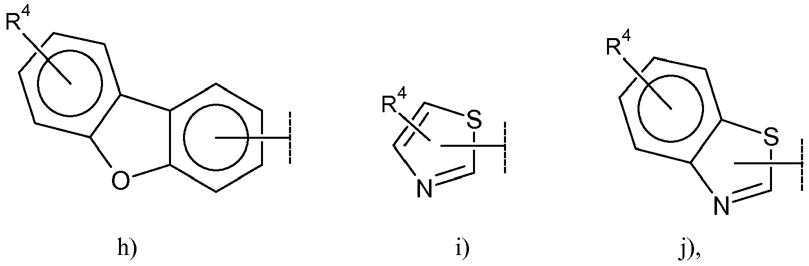 und wobei R⁴ cyclischer oder acyclischer, substituierter oder unsubstituierter, verzweigter oder unverzweigter Aliphat; cyclischer oder acyclischer, substituierter oder unsubstituierter, verzweigter oder unverzweigter Heteroaliphat; substituiertes oder unsubstituiertes, verzweigtes oder unverzweigtes Acyl; substituiertes oder unsubstituiertes, verzweigtes oder unverzweigtes Aryl; substituiertes oder unsubstituiertes, verzweigtes oder unverzweigtes Heteroaryl ist.

7. Verfahren zur Herstellung einer Verbindung nach einem der Ansprüche 1 bis 6, wobei R¹ eine Hydroxamsäuregruppe CONHOH ist, umfassend die Schritte des
(1) Inreaktionbringens einer perfluorierten Verbindung (III) 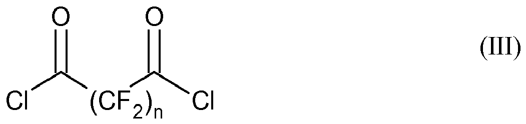 mit einem primären, sekundären oder tertiären Alkohol RₓOH, wobei Rₓ ein cyclischer oder acyclischer, substituierter oder unsubstituierter, verzweigter oder unverzweigter Aliphat oder Aromat ist,
(2) Inreaktionbringens des Produkts (IV) von Schritt (1) 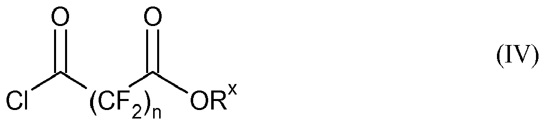 mit RNH₂, und
(3) Inreaktionbringens des Produkts (V) von Schritt (2) 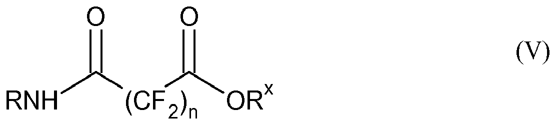 mit H₂NOH.

8. Pharmazeutische Zusammensetzung umfassend eine Verbindung nach einem der Ansprüche 1 bis 6 und einen pharmazeutisch annehmbaren Träger.

9. Verbindung nach einem der Ansprüche 1 bis 6 zur Verwendung als Arzneimittel.

10. Verbindung nach einem der Ansprüche 1 bis 6 zur Verwendung als Arzneimittel zur Behandlung von Krebs.

## Revendications

1. Composé inhibiteur de l'histone désacétylase (HDAC) de formule (I) 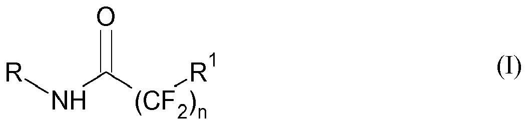 ou un sel pharmaceutiquement acceptable de celui-ci, dans lequel
n vaut 4, 5, 6, 7, 8, 9, 10, 11, 12, 13 ou 14,
R est un groupe aliphatique ramifié ou non ramifié, substitué ou non substitué, cyclique ou acyclique; un groupe hétéroaliphatique ramifié ou non ramifié, substitué ou non substitué, cyclique ou acyclique; un groupe acyle ramifié ou non ramifié, substitué ou non substitué; un groupe aryle ramifié ou non ramifié, substitué ou non substitué; un groupe hétéroaryle ramifié ou non ramifié, substitué ou non substitué, et
R¹ est un groupe de liaison métallique capable de lier un ion métallique à l'intérieur d'un site actif de ladite histone désacétylase.

2. Composé selon la revendication 1, dans lequel n vaut 4, 5, 6, 7 ou 8, de préférence de 5, 6 ou 7, de manière davantage préférée 6 ou 7, et de manière spécialement préférée 6.

3. Composé selon la revendication 1 ou 2, dans lequel R¹ est l'un des suivants

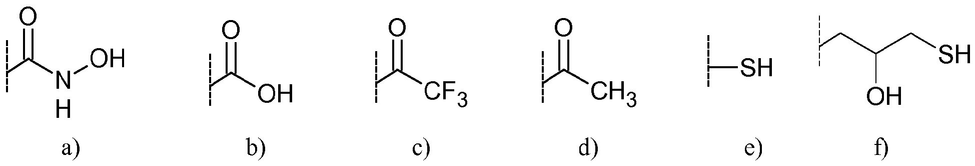

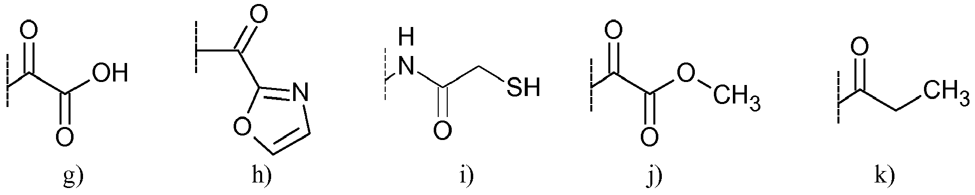

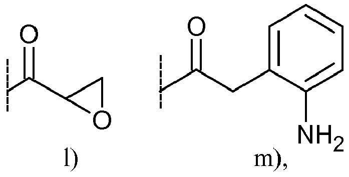

4. Composé selon l'une des revendications précédentes, dans lequel
R est 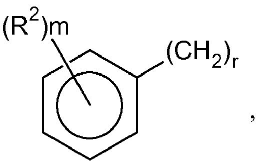 dans lequel
m vaut 0, 1, 2, 3, 4 ou 5
r vaut 0, 1, 2 ou 3, et dans lequel
R² est d'hydrogène d'halogène; un groupe aliphatique ramifié ou non ramifié, substitué ou non substitué, cyclique ou acyclique; un groupe hétéroaliphatique ramifié ou non ramifié, substitué ou non substitué, cyclique ou acyclique; un groupe acyle ramifié ou non ramifié, substitué ou non substitué; un groupe aryle ramifié ou non ramifié, substitué ou non substitué; un groupe hétéroaryle ramifié ou non ramifié, substitué ou non substitué.

5. Composé selon la revendication 4, dans lequel R² est l'un des suivants: 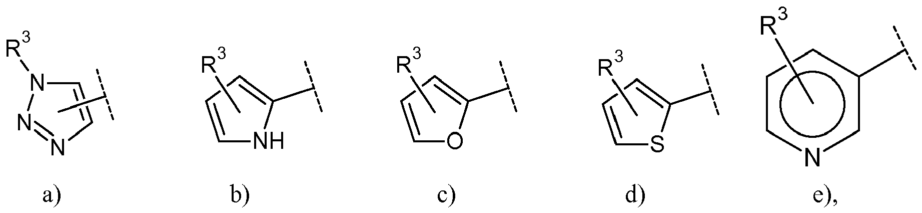 et dans lequel R³ est d'hydrogène; d'halogène; un groupe aliphatique ramifié ou non ramifié, substitué ou non substitué, cyclique ou acyclique; un groupe hétéroaliphatique ramifié ou non ramifié, substitué ou non substitué, cyclique ou acyclique; un groupe acyle ramifié ou non ramifié, substitué ou non substitué; un groupe aryle ramifié ou non ramifié, substitué ou non substitué; un groupe hétéroaryle ramifié ou non ramifié, substitué ou non substitué.

6. Composé selon la revendication 1, dans lequel R est l'un des suivants: 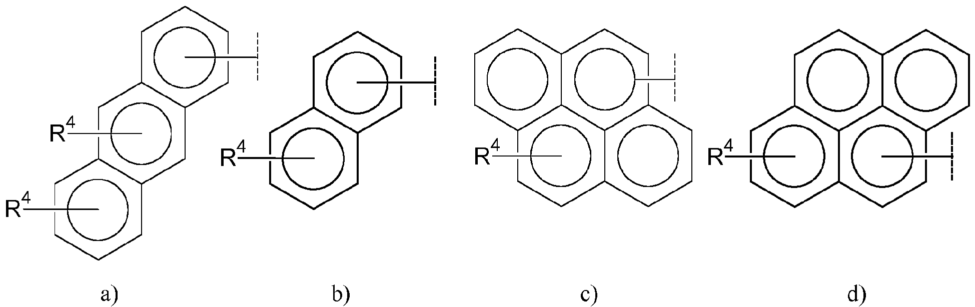 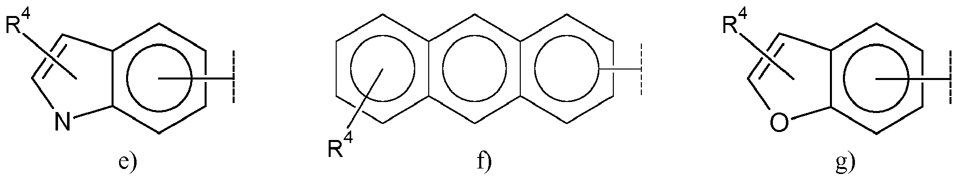 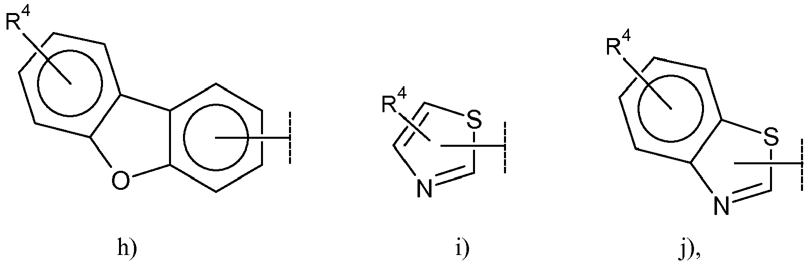 et dans lequel R⁴ est un groupe aliphatique ramifié ou non ramifié, substitué ou non substitué, cyclique ou acyclique; un groupe hétéroaliphatique ramifié ou non ramifié, substitué ou non substitué, cyclique ou acyclique; un groupe acyle ramifié ou non ramifié, substitué ou non substitué; un groupe aryle ramifié ou non ramifié, substitué ou non substitué; un groupe hétéroaryle ramifié ou non ramifié, substitué ou non substitué.

7. Procédé de fabrication d'un composé selon l'une des revendications 1 à 6, R1 étant un groupe acide hydroxamique CONHOH, comprenant les étapes de
(1) Mise en réaction d'un composé perfluoré (III), 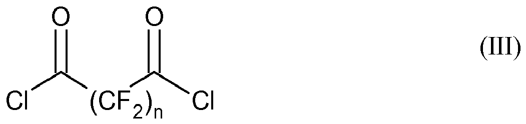 avec un alcool primaire, secondaire ou tertiaire RₓOH, dans lequel Rₓ est un groupe aliphatique ou aromatique ramifié ou non ramifié, substitué ou non substitué, cyclique ou acyclique,
(2) Mise en réaction du produit (IV) de l'étape (1) 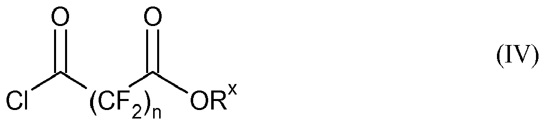 avec RNH₂, et
(3) Mise en réaction du produit (V) de l'étape (2) 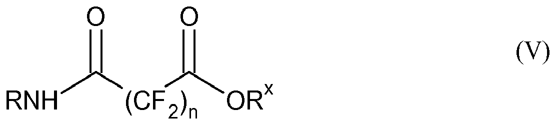 avec H₂NOH.

8. Composition pharmaceutique comprenant un composé selon l'une quelconque des revendications 1 à 6, et un vecteur pharmaceutiquement acceptable.

9. Composé de l'une des revendications 1 à 6, pour son utilisation en tant que médicament.

10. Composé de l'une des revendications 1 à 6, pour son utilisation en tant que médicament pour le traitement du cancer.
